# EUROPÄISCHE PATENTSCHRIFT

(11) **EP 3 377 124 B1**
(45) Veröffentlichungstag und Bekanntmachung des Hinweises auf die Patenterteilung: **09.09.2020**
(21) Anmeldenummer: 16795323.1
(22) Anmeldetag: 14.11.2016
(51) Int. Cl.: A61L 27/26, A61L 27/38, A61L 27/60, C12N 5/071, G01N 33/50

(54) **VERFAHREN ZUR HERSTELLUNG EINES HAUTÄQUIVALENTS SOWIE DESSEN VERWENDUNG FÜR IN VITRO TESTS UND IN VIVO TRANSPLANTATE**
METHOD FOR PRODUCING A SKIN EQUIVALENT, AND USE THEREOF FOR IN VITRO TESTS AND IN VIVO TRANSPLANTS
PROCÉDÉ DE FABRICATION D'UN ÉQUIVALENT DE PEAU ET SON UTILISATION POUR DES ESSAIS IN VITRO ET DES GREFFES IN VIVO

(30) Priorität: 17.11.2015 DE 102015119877
(43) Veröffentlichungstag der Anmeldung: 26.09.2018
(73) Patentinhaber: Technische Universität Berlin, 10623 Berlin (DE)
(72) Erfinder: LINDNER, Gerd, 13469 Berlin (DE)
(74) Vertreter: Gulde & Partner
(86) Internationale Anmeldenummer: PCT/EP2016/077539
(87) Internationale Veröffentlichungsnummer: WO 2017/084997

(56) Entgegenhaltungen:
- EP-A1- 0 285 471

## Beschreibung

Die Haut ist die Schnittstelle zwischen dem Körper und seiner Umgebung. Sie ist die erste entscheidende Barriere für potentiell schädliche äußere Einflüsse wie z.B. mechanische Beanspruchung oder Invasion von pathogenen Mikroorganismen. Die essentielle Bedeutung der Haut für die menschliche Gesundheit wird insbesondere dann deutlich, wenn die selbstregenerative Kapazität nicht mehr ausreicht um diesen Schutz zu erhalten. Dies ist insbesondere bei Patienten der Fall, die an chronischen oder großflächigen akuten Wunden leiden, wie sie bei Verletzungen wie z.B. Verbrennungen auftreten. Werden diese Wunden nicht ausreichend mit Therapien zur Hautwiederherstellung behandelt, haben diese Bedingungen erhebliche Auswirkungen auf die Lebensqualität und können sogar zum Tod führen.

Neben den Möglichkeiten zur Wiederherstellung verletzter Haut können künstlich erzeugte Hautäquivalente, welche u.a. ebenfalls Haarfollikel umfassen, auch zur therapeutischen Behandlung der unterschiedlichsten Formen von Haarausfall, sogenannter Alopezien, verwendet werden.

Zudem besteht ein Bedarf an einem geeigneten Testsystem, welches für die Erforschung der Ursachen der verschiedenen Arten von Alopezie verwendet werden kann. Gleiches gilt für Untersuchungen von Wachstum, der Struktur und der Pigmentierung der Haare. Neben der biomedizinischen Forschung verzeichnet insbesondere die pharmazeutische und kosmetische Industrie einen dringenden Bedarf an geeigneten Systemen für die Testung der Wirksamkeit oder Toxizität von Substanzen auf die menschliche Haut bzw. die Haare.

In den vergangenen Jahrzehnten wurden große Anstrengungen unternommen die komplexen Strukturen und die vielschichtigen Funktionen der menschlichen Haut in Form von Hautersatz zu imitieren. So wird beispielsweise in EP 0 285 471 A die Bereitstellung eines mehrschichtigen Hautäquivalents beschrieben.

Trotz weitreichender Fortschritte bei der Entwicklung künstlicher Haut, stellen die immer noch sehr stark abweichende Struktur und Funktion zur Physiologie der natürlichen menschlichen Haut ein Problem dar. Besonders die Implementierung von Haarfollikeln, Fettgewebe und einer vaskulären Versorgung der künstlichen Haut ist bisher nur ungenügend realisiert worden. Weiterhin kann die Herstellung einer solchen künstlichen Haut bisher nicht in großem Maßstab erfolgen, da Hochdurchsatzverfahren für derartige Produktionen derzeit noch limitiert sind.

Aufgabe der vorliegenden Erfindung ist es, einen oder mehrere Nachteile des Standes der Technik zu vermindern oder zu vermeiden. Insbesondere ist es eine Aufgabe der vorliegenden Erfindung ein Verfahren zur Verfügung zu stellen, mit dem Hautäquivalente hergestellt werden können.

Die vorliegende Erfindung löst diese Aufgabe durch Bereitstellung eines Verfahrens zur Herstellung eines Hautäquivalents, umfassend die Schritte: a) Herstellen einer dermalen Matrix enthaltend eine erste Schicht und eine zweite Schicht, umfassend die Schritte: i) Bereitstellen der ersten Schicht; ii) Bereitstellen einer polymerisierbaren Lösung umfassend eine Flüssigkeit und mindestens ein Polymer ausgewählt aus Kollagen, Elastin und/oder Hyaluron; iii) Hinzufügen der polymerisierbaren Lösung zur ersten Schicht; iv) Polymerisierung der Lösung zur zweiten Schicht; v) Komprimieren der zweiten Schicht, wobei durch das Komprimieren der Flüssigkeitsgehalt der zweiten Schicht reduziert wird und die erste Schicht und die zweite Schicht zur dermalen Matrix zusammengefügt werden; und vi) Einbringen von mindestens einer Kavität in die dermale Matrix; b) Einbringen von mindestens einem Haarfollikel und/oder Haarfollikelkeim in die mindestens eine Kavität der in Schritt a) hergestellten dermalen Matrix zur Herstellung eines Hautsurrogats; und c) Hinzufügen von mindestens einer weiteren Zellpopulation ausgewählt aus Fibroblasten, Endothelzellen, Adipozyten, Nervenzellen, Drüsenzellen, Melanozyten oder Keratinozyten zum Hautsurrogat zur Herstellung des Hautäquivalents.

Das Herstellungsverfahren der vorliegenden Erfindung wird in vitro durchgeführt. Als "in vitro" wird jede Umgebung verstanden, die sich nicht innerhalb eines lebenden Organismus, z.B. einem menschlichen oder tierischen Körpers, befindet. Das erfindungsgemäße in vitro Nachweisverfahren umfasst also ausdrücklich kein Verfahren, welches am menschlichen oder tierischen Körper vorgenommen wird.

Soweit nachfolgend der Kontext nicht eindeutig etwas anderes ergibt, ist bei der Verwendung von Singular-Formen bzw. Plural-Formen stets sowohl die Ein- als auch die Mehrzahl umfasst.

Das erfindungsgemäße Verfahren bezieht sich auf die Herstellung eines Hautäquivalents. Der Begriff "Hautäquivalent" bezeichnet ein Hautmodell, welches als ein im Wesentlichen gleichwertiger Ersatz für eine natürliche Haut bezüglich der Beschaffenheit und Funktion verwendet werden kann. Vorzugsweise weist das Hautäquivalent die ungefähre Form und Struktur einer physiologischen Haut auf.

Das erfindungsgemäße Verfahren zur Herstellung eines Hautäquivalents umfasst die Schritte a) Herstellen einer dermalen Matrix enthaltend eine erste Schicht und eine zweite Schicht; b) Einbringen von mindestens einem Haarfollikel und/oder Haarfollikelkeim in die mindestens eine Kavität der in Schritt a) hergestellten dermalen Matrix zur Herstellung eines Hautsurrogats und c) Hinzufügen von mindestens einer weiteren Zellpopulation ausgewählt aus Fibroblasten, Endothelzellen, Adipozyten, Nervenzellen, Drüsenzellen, Melanozyten oder Keratinozyten zum Hautsurrogat zur Herstellung des Hautäquivalents.

Beim erfindungsgemäßen Verfahren zur Herstellung eines Hautäquivalents wird in einem ersten Schritt a) eine dermale Matrix hergestellt. Die Herstellung der dermalen Matrix umfasst die Schritte: i) Bereitstellen der ersten Schicht; ii) Bereitstellen einer polymerisierbaren Lösung umfassend eine Flüssigkeit und mindestens ein Polymer ausgewählt aus Kollagen, Elastin und/oder Hyaluron; iii) Hinzufügen der polymerisierbaren Lösung zur ersten Schicht; iv) Polymerisierung der Lösung zur zweiten Schicht; v) Komprimieren der zweiten Schicht, wobei durch das Komprimieren der Flüssigkeitsgehalt der zweiten Schicht reduziert wird und die erste Schicht und die zweite Schicht zur dermalen Matrix zusammengefügt werden; und vi) Einbringen von mindestens einer Kavität in die dermale Matrix.

Die dermale Matrix enthält zwei Schichten, eine erste Schicht und eine zweite Schicht. Als "dermale Matrix" wird vorliegend ein stoffliches Substrat verstanden, das im erfindungsgemäßen Verfahren als Ausgangsmaterial für die Herstellung des Hautäquivalents verwendet wird. Die dermale Matrix weist dabei Strukturähnlichkeiten zum schichtartigen Aufbau einer physiologischen Haut auf, so ähnelt die erste Schicht der (sub)dermalen und die zweite Schicht der papillären und retikulären Struktur physiologischer Haut.

In Schritt i) wird die erste Schicht der dermalen Matrix bereitgestellt. Vorzugsweise enthält die erste Schicht der dermalen Matrix im Wesentlichen keine lebenden Zellen.

Die erste Schicht der dermalen Matrix kann eine natürliche Hautsubstanz umfassen. Unter "Hautsubstanz" wird ein Material verstanden, welches der bindegewebsartigen Struktur der (Sub)Dermis einer physiologischen Haut ähnelt oder diese Struktur aufweist. Bevorzugt ist die natürliche Hautsubstanz vor ihrer Verwendung im erfindungsgemäßen Verfahren dezellularisiert worden. Beispielsweise kann es sich bei der natürlichen Hautsubstanz um eine Spenderhaut handeln. Diese Spenderhaut ist tierischen Ursprungs. Vorzugsweise stammt die Spenderhaut aus einem Wirbeltier, welches ein Säugetier, Vogel, Reptil oder eine Amphibie sein kann, bevorzugt ein Vogel oder Säugetier. Dieses Säugetier kann ein Mensch, Affe, Schwein, Hund, Katze oder Nagetier sein, bevorzugt ein Mensch. Eine Spenderhaut humanen Ursprungs ist z.B. unter dem Namen Epiflex® im Stand der Technik bekannt.

Die Spenderhaut kann dabei aus jeder geeigneten Körperregion stammen, beispielsweise aus dem Rumpfbereich wie z.B. aus Brust, Bauch oder Rücken, oder aus den Gliedmaßen wie z.B. aus Armen oder Beinen.

Nachdem die Haut dem Spender entnommen wurde, kann diese Haut einem oder mehreren Reinigungsschritten unterzogen werden, um möglicherweise vorhandene störende

Substanzen von der Haut zu entfernen. Ein derartiger Reinigungsschritt kann z.B. die Beseitigung von lebenden Zellen umfassen.

Die erste Schicht der dermalen Matrix kann eine künstliche Hautsubstanz umfassen. Beispielsweise kann es sich bei der künstlichen Hautsubstanz um ein Material handeln, welches Strukturproteine umfasst, vorzugsweise Kollagen und/oder Elastin, besonders bevorzugt Kollagen und Elastin. Eine künstliche Hautsubstanz ist z.B. unter dem Namen Matriderm® im Stand der Technik bekannt.

Im nachfolgenden Schritt ii) wird eine polymerisierbare Lösung bereitgestellt. Diese polymerisierbare Lösung ist geeignet in einer Polymerisationsreaktion vom flüssigen in einen im Wesentlichen festen Zustand umgewandelt zu werden. Die polymerisierbare Lösung umfasst eine Flüssigkeit. Bei der Flüssigkeit kann es sich um z.B. um Wasser, eine Salzlösung oder ein Nährmedium handeln. Die Flüssigkeit kann gepuffert sein.

Die polymerisierbare Lösung weist vorzugsweise einen pH-Wert von 2 bis 12 auf, bevorzugt von 4 bis 10, besonders bevorzugt von 5 bis 9, ganz besonders bevorzugt von 6 bis 8.

Die polymerisierbare Lösung umfasst weiterhin mindestens ein Polymer ausgewählt aus Kollagen, Elastin und/oder Hyaluron. Bevorzugt umfasst die polymerisierbare Lösung Kollagen. Vorzugsweise handelt es sich bei dem Kollagen um ein Kollagen des Typs I, II, III, IV oder V, bevorzugt um Kollagen des Typs I. Ebenfalls bevorzugt umfasst die polymerisierbare Lösung Kollagen, Elastin und Hyaluron.

Das mindestens eine Polymer ist in der polymerisierbaren Lösung in einer Konzentration von ≥0,01 % enthalten, bevorzugt von 0,01% bis 50%, besonders bevorzugt 0,1% bis 10%, ganz besonders bevorzugt 0,125% bis 5%.

Die polymerisierbare Lösung kann zusätzlich Zellen enthalten. Vorzugsweise handelt es sich dabei um lebende Zellen. Bei den Zellen kann es sich um solche mesenchymalen Ursprungs handeln wie z.B. multipotente mesenchymale Stammzellen, welche Vorläuferzellen des Bindegewebes darstellen. Vorzugsweise handelt es sich bei den Zellen um Bindegewebszellen, besonders bevorzugt um Fibroblasten.

Im nachfolgenden Schritt iii) wird die polymerisierbare Lösung zur ersten Schicht hinzugefügt.

Im folgenden Schritt iv) erfolgt die Polymerisierung der Lösung zur zweiten Schicht der dermalen Matrix. Dies findet bevorzugt bei einer Temperatur von ≥ Raumtemperatur statt. Als "Raumtemperatur" wird eine Temperatur im Bereich von 18°C bis 25°Cbestimmt. Vorzugsweise findet die Polymerisierung der Lösung bei einer Temperatur von ≥25°C statt, bevorzugt bei ≥30°C, besonders bevorzugt bei 30°C bis 50°C, ganz besonders bevorzugt bei 35°C bis 45°C, insbesondere bevorzugt bei 37°C bis 42°C.

Die Polymerisierung der Lösung zur zweiten Schicht kann unter im Wesentlichen statischen Bedingungen oder unter Bewegung stattfinden. Vorzugsweise findet die Polymerisierung unter Rotations-, Schwenk- oder Schüttelbewegungen statt. Ebenso kann die Polymerisierung der Lösung zur zweiten Schicht unter Bedingungen stattfinden, welche beide ebengenannten Methoden kombiniert, so dass die Polymerisierungsreaktion beispielsweise zunächst unter Bewegung initiiert und dann unter im Wesentlichen statischen Bedingungen finalisiert wird.

Die Polymerisierung der Lösung zur zweiten Schicht findet für mindestens 1 min statt, bevorzugt für 2 min bis 24h, besonders bevorzugt für 5 min bis 12h, besonders bevorzugt für 10 min bis 4h, ganz besonders bevorzugt für 15 min bis 1h.

Optional kann vor Schritt iv) das Polymer in der polymerisierbaren Lösung durch Bewegung strukturiert werden. Die Strukturierung des Polymers bewirkt eine gleichmäßige Verteilung des Polymers in der Lösung, so dass die daraus entstehende polymerisierte zweite Schicht eine im Wesentlichen gleichförmige Struktur aufweist.

Im nachfolgenden Schritt v) erfolgt das Komprimieren der zweiten Schicht, wobei durch das Komprimieren der Flüssigkeitsgehalt der zweiten Schicht reduziert wird und die erste Schicht und die zweite Schicht zur dermalen Matrix zusammengefügt werden. Dabei kann das Komprimieren der zweiten Schicht durch Zentrifugieren, Sedimentation oder mechanische Krafteinwirkung erfolgen. Vorzugsweise handelt es sich bei der mechanischen Krafteinwirkung um ein Pressen. Ein Teil des Flüssigkeitsgehalts, welcher durch das Komprimieren aus der zweiten Schicht entfernt wird, kann dabei von der ersten Schicht aufgenommen werden.

Durch das Komprimieren verbinden sich die erste und die zweite Schicht zur dermalen Matrix. Die dermale Matrix weist eine Schichtdicke von mindestens 0,1 mm auf, bevorzugt 0,25mm bis 10mm, besonders bevorzugt 0,5mm bis 5mm, ganz besonders bevorzugt 1 mm bis 3mm. Dabei können die erste Schicht und die zweite Schicht innerhalb der dermalen Matrix eine gleiche oder unterschiedliche jeweilige Schichtdicke aufweisen. Die erste Schicht weist eine Schichtdicke von mindestens 0,01 mm auf, bevorzugt 0,05mm bis 5mm, besonders bevorzugt 0,1 mm bis 2,5mm. Die zweite Schicht weist eine Schichtdicke von mindestens 0,01mm auf, bevorzugt 0,05mm bis 5mm, besonders bevorzugt 0,1mm bis 2,5mm.

Im nächsten Schritt vi) erfolgt das Einbringen von mindestens einer Kavität in die dermale Matrix. Die Kavitäten der dermalen Matrix weisen einen mittleren Durchmesser von ≥0,001 mm bis 5 mm auf, bevorzugt von 0,005mm bis 1mm, besonders bevorzugt von 0,01mm bis 0,08mm, ganz besonders bevorzugt von 0,02mm bis 0,06mm, insbesondere bevorzugt von 0,05mm bis 0,04mm. Die Kavitäten der dermalen Matrix weisen eine Tiefe von ≥0,05mm bis 10 mm auf, bevorzugt von 0,1mm bis 8mm, besonders bevorzugt von 0,15mm bis 6mm, ganz besonders bevorzugt von 0,2mm bis 4mm, insbesondere bevorzugt von 0,5mm bis 2,5mm.

Vorzugsweise werden 1 bis 400 Kavitäten/cm² in die dermale Matrix eingebracht, bevorzugt 10 bis 200/cm², besonders bevorzugt 20 bis 100/ cm², ganz besonders bevorzugt 30 bis 100/ cm². Dabei können die Kavitäten in regelmäßigen oder in unregelmäßigen Abständen in die dermale Matrix eingebracht werden, vorzugsweise werden die Kavitäten in regelmäßigen Abständen in die dermale Matrix eingebracht.

Das Einbringen der mindestens einen Kavität kann durch Entfernung von Material der dermalen Matrix erfolgen. Vorzugsweise wird die mindestens eine Kavität durch Einstechen, Ausschneiden oder mittels einer Abdruckform in die dermale Matrix eingebracht. Das Einstechen erfolgt beispielsweise mithilfe einer Nadel, insbesondere einer Hohlnadel. Das Ausschneiden kann z.B. mittels eines Skalpells erfolgen, gegebenenfalls unter Zuhilfenahme weiterer Werkzeuge wie z.B. einer Pinzette, um das herausgeschnittene Material aus der dermalen Matrix zu entfernen.

Alternativ kann die mindestens eine Kavität mittels einer Abdruckform in die dermale Matrix eingebracht werden. Die Abdruckform umfasst eine Basis. Die Basis kann verschiedenartig geformt sein, so kann sie beispielsweise eine runde, ovale oder eckige Grundfläche aufweisen. Die Basis kann planar oder strukturiert sein. Vorzugsweise umfasst die Abdruckform mindestens einen Zylinder. Unter dem Begriff "Zylinder" wird ein länglicher zylindrischer Körper verstanden, welcher geeignet ist eine Kavität in die dermale Matrix einzubringen. Der mindestens eine Zylinder weist einen Durchmesser von ≥0,001 mm bis 5mm auf, bevorzugt von 0,005mm bis 1mm, besonders bevorzugt von 0,01mm bis 0,08mm aufweisen, ganz besonders bevorzugt von 0,02mm bis 0,06mm, insbesondere bevorzugt von 0,05mm bis 0,04mm. Der mindestens eine Zylinder weist eine Länge von ≥0,05mm bis 10mm auf, bevorzugt von 0,1mm bis 8mm, besonders bevorzugt von 0,15mm bis 6mm, ganz besonders bevorzugt von 0,2mm bis 4mm, insbesondere bevorzugt von 0,5mm bis 2,5mm.

Vorzugsweise ist der mindestens eine Zylinder an der Basis befestigt, besonders bevorzugt ist der mindestens eine Zylinder in der Basis integriert. Bevorzugt ist der mindestens eine Zylinder senkrecht zur Grundfläche der Basis ausgerichtet.

Vorzugsweise umfasst die Abdruckform ≥1 Zylinder. Bevorzugt umfasst die Abdruckform 10 bis 10000 Zylinder, besonders bevorzugt 20 bis 5000, ganz besonders bevorzugt 100 bis 1000. Die Zylinder können in regelmäßigen oder in unregelmäßigen Abständen in der Abdruckform vorhanden sein. Vorzugsweise sind die Zylinder in regelmäßigen Abständen in der Abdruckform vorhanden.

Vorzugsweise weist der mindestens eine Zylinder zumindest teilweise einen Hohlraum auf, besonders bevorzugt ist der Zylinder im Wesentlichen vollständig hohl.

Der mindestens eine Zylinder kann an einem seiner Enden eine trichterförmige Aufweitung aufweisen. In diesem Fall ist der Zylinder bevorzugt im Wesentlichen vollständig hohl. Die trichterförmige Aufweitung des Zylinders weist an ihrem äußeren Ende einen Durchmesser von ≥0,01 mm auf, bevorzugt von 0,1mm bis 4mm, besonders bevorzugt von 0,2mm bis 3mm aufweisen, ganz besonders bevorzugt von 0,3mm bis 2,5mm, insbesondere bevorzugt von 0,5mm bis 2mm.

Ebenfalls bevorzugt umfasst die Abdruckform mindestens eine Erhebung. Der Ausdruck "Erhebung" bezeichnet eine Erhöhung, welche im Wesentlichen kegelförmig geformt ist. Bevorzugt weist die Erhebung die Form eines geraden Kegels auf, wobei die Spitze des Kegels vorzugsweise abgerundet ist. Mithilfe dieser mindestens einen Erhebung wird eine wellenartige Struktur in die dermale Matrix eingeprägt, welche der physiologischen Papillenstruktur von natürlicher Haut an der Epidermis/Dermis-Grenze ähnelt. Die Grundfläche der kegelförmigen Erhebung weist einen Durchmesser von ≥0,01 mm auf, bevorzugt von 0,1mm bis 4mm, besonders bevorzugt von 0,2mm bis 3mm aufweisen, ganz besonders bevorzugt von 0,3mm bis 2,5mm, insbesondere bevorzugt von 0,5mm bis 2mm. Die abgerundete Kegelspitze weist einen Durchmesser von ≥0,001 mm auf, bevorzugt von 0,01mm bis 3,5mm, besonders bevorzugt von 0,1mm bis 2,5mm aufweisen, ganz besonders bevorzugt von 0,2mm bis 2mm, insbesondere bevorzugt von 0,3mm bis 1mm.

Vorzugsweise ist die mindestens eine Erhebung an der Basis befestigt, besonders bevorzugt ist die mindestens eine Erhebung in der Basis integriert. Ebenso ist es möglich, dass es sich bei der mindestens einen Erhebung um eine strukturgebende Einheit handelt, welche einer strukturierten Basis eine entsprechende Form verleiht. Bevorzugt ist die Höhe der mindestens einen Erhebung senkrecht zur Grundfläche der Basis ausgerichtet.

Vorzugsweise umfasst die Abdruckform ≥1 Erhebung. Bevorzugt umfasst die Abdruckform 10 bis 10000 Erhebungen, besonders bevorzugt 20 bis 5000, ganz besonders bevorzugt 100 bis 1000. Die Erhebungen können in regelmäßigen oder in unregelmäßigen Abständen in der Abdruckform vorhanden sein. Vorzugsweise sind die Erhebungen in regelmäßigen Abständen in der Abdruckform vorhanden.

Vorzugsweise umfasst die Abdruckform mindestens einen Zylinder und mindestens eine Erhebung. Bevorzugt umfasst die Abdruckform 10 bis 10000 Zylinder und Erhebungen, besonders bevorzugt 20 bis 5000, ganz besonders bevorzugt 100 bis 1000. Die Zylinder und/oder Erhebungen können jeweils in regelmäßigen oder in unregelmäßigen Abständen in der Abdruckform vorhanden sein. Vorzugsweise sind die Zylinder und/oder Erhebungen in regelmäßigen Abständen in der Abdruckform vorhanden, bevorzugt sind die Zylinder und die Erhebungen in regelmäßigen Abständen in der Abdruckform vorhanden. Vorzugsweise sind die Zylinder und Erhebungen derart in der Abdruckform vorhanden, dass sie im Wesentlichen versetzt zueinander sind.

Die Basis der Abdruckform, der mindestens eine Zylinder und/oder die mindestens eine Erhebung können beispielsweise aus Metall, Plastik, Kautschuk oder Silikon wie z.B. Polydimethylsiloxan (PDMS) bestehen oder diese Materialien umfassen. Vorzugsweise bestehen die Basis der Abdruckform, der mindestens eine Zylinder und die mindestens eine Erhebung aus demselben Material. Besonders bevorzugt sind die Basis, der mindestens eine Zylinder und die mindestens eine Erhebung im Wesentlichen als einstückige Abdruckform ausgebildet.

Die Verfahrensschritte iv), v) oder vi) zur Herstellung der dermalen Matrix können nacheinander und/oder gleichzeitig stattfinden. Vorzugsweise erfolgt das Einbringen der mindestens einen Kavität in die dermale Matrix in Schritt vi) während oder nach der Polymerisierung in Schritt iv). Besonders bevorzugt erfolgen die Schritte iv) und vi) gleichzeitig, so dass also das Einbringen der mindestens einen Kavität in die dermale Matrix vorzugsweise während der Polymerisierung erfolgt. Insbesondere bevorzugt erfolgen die Schritte iv) und vi) gleichzeitig, wobei die Polymerisierung vorzugsweise unter Bewegung stattfindet, besonders bevorzugt unter Rotations-, Schwenk- oder Schüttelbewegungen.

Nach der Herstellung der dermalen Matrix in Schritt a), wird in Schritt b) des erfindungsgemäßen Verfahrens mindestens ein Haarfollikel und/oder Haarfollikelkeim in die mindestens eine Kavität der dermalen Matrix zur Herstellung eines Hautsurrogats eingebracht. Vorzugsweise werden eine, mehrere oder alle Kavitäten der dermalen Matrix vor dem Einbringen des Haarfollikels und/oder Haarfollikelkeims in Schritt b) mit extrazellulären Matrixproteinen beschichtet wird.

Der Begriff "Haarfollikel" bezeichnet natürliche Strukturen, welche die Haarwurzel umgeben und dadurch das Haar in der Haut verankern. Der Haarfollikel umfasst im Querschnitt zwei Schichten, nämlich eine äußere und eine innere epitheliale Haarwurzelscheide. Die äußere epitheliale Haarwurzelscheide stellt eine trichterartige Einstülpung der Basalschicht der Haut dar, welche eine Hülle für die Haarwurzel bildet. Die innere epitheliale Haarwurzelscheide umfasst die Haarwurzel und besteht aus drei Schichten, nämlich einer inneren Schicht (Kutikula), einer mittleren Schicht (Huxley-Schicht) und einer äußeren Schicht (Henle-Schicht). In den Haarfollikel münden Drüsen, die Talg und andere Substanzen wie z.B. Duftstoffe produzieren und aussondern. In der Nähe der Talgdrüsen setzen ebenfalls Haarbalgmuskeln, die Musculi arrectores pilorum, am Haarfollikel an, welche die Haare aufstellen können und für die sogenannte Gänsehaut verantwortlich sind. Ferner enden feine Nervenfasern im Haarfollikel, welche dem Tastsinn und der Steuerung der Haarbalgmuskeln dienen. Erfindungsgemäß umfasst der Begriff "Haarfollikel" ebenfalls eine im Vergleich zu natürlichen Mikrofollikeln unvollständige Mikrofollikel-Struktur, welche die äußere und/oder innere epitheliale Haarwurzelscheide umfassen kann, allerdings ohne weitere Zelltypen wie Muskel- oder Nervenzellen, Blutgefäße etc. zu enthalten, so dass der Haarfollikel eine verringerte Größe im Vergleich zu natürlichen Haarfollikeln aufweist. Ein Haarfollikel weist eine dreidimensionale, gegebenenfalls räumlich begrenzte Form auf, welche dem dreidimensionalen Erscheinungsbild eines physiologischen Haarfollikels ähnelt. Die Haarfollikel können eine polare Struktur aufweisen, beispielsweise eine spitze Auswölbung auf einer Seite des Haarfollikels, die an Strukturen der frühen Haarmorphogenese erinnern.

Bei dem verwendeten Haarfollikel kann es sich also um einen aus einem Gewebe isolierten Haarfollikel handeln. Dem Fachmann sind Verfahren zur Entnahme von Haarfollikeln aus Gewebe bekannt. Bevorzugt werden die Haarfollikel nach Standards guter medizinsicher Praxis aus dem Gewebe entnommen. Vorzugsweise erfolgt die Entnahme der Haarfollikel aus dem Gewebe derart, dass sie im Wesentlichen intakt, d.h. unbeschädigt, bleiben. Die Entnahme der Haarfollikel aus dem Gewebe kann beispielsweise wie folgt durchgeführt werden: i) Abtrennung der Epidermis von der darunterliegenden Dermis bzw. Fettgewebe beim Gewebe, vorzugsweise unter Verwendung eines Skalpells; ii) Präparation von Haaren aus der Dermis bzw. dem Fettgewebe des Gewebes. Die entnommenen Haare umfassen an ihrem proximalen Ende die Haarfollikel. Optional kann vor Schritt ii) die Dermis bzw, das Fettgewebe komprimiert werden, damit die darin vorhandenen Haarfollikel leichter herauspräpariert werden können. Das Komprimieren kann dabei beispielsweise mithilfe einer Pinzette durchgeführt werden. Vorzugsweise findet die Entnahme der Haarfollikel unter dem Mikroskop statt.

Das Gewebe aus dem die Haarfollikel gewonnen werden können stammt aus einem Säugetier. Dieses Säugetier kann ein Mensch, Affe, Schwein, Hund, Katze oder Nagetier sein, bevorzugt ein Mensch. Das Säugetier stellt also einen Spender für das, den Haarfollikel enthaltene, Gewebe dar.

Das Gewebe umfasst bevorzugt Haut. Dabei kann das Gewebe aus jeder behaarten Körperregion gewonnen werden vorzugsweise aus Kopf, Augenbrauen, Bart, Genitalbereich, Bein oder anderen Körperregionen. Vorzugsweise wird das Gewebe mittels einer Biopsie erhalten.

Bevor die Haarfollikel aus dem Gewebe entnommen werden, kann dieses Gewebe einem oder mehreren Reinigungsschritten unterzogen werden, um möglicherweise vorhandene störende Substanzen von dem Gewebe zu entfernen.

Vorzugsweise werden allogene Haarfollikel für das Hautäquivalent verwendet, d.h. der Spender des Gewebes, aus dem die Haarfollikel isoliert werden, und der Empfänger des Hautäquivalents, welches die isolierten Haarfollikel umfasst, der gleichen Art angehören, dass also z.B. sowohl Spender als auch Empfänger Menschen sind. Besonders bevorzugt ist es autologe Haarfollikel für das Hautäquivalent zu verwenden, d.h. dass der Spender des Gewebes, aus dem die Haarfollikel isoliert werden, und der Empfänger des Hautäquivalents, welches die isolierten Haarfollikel umfasst, dieselbe Person ist, z.B. derselbe Mensch.

Ebenso ist es möglich, dass die Haarfollikelbildung in den Kavitäten der dermalen Matrix erfolgt. Dafür wird in Schritt b) des erfindungsgemäßen Verfahrens mindestens ein Haarfollikelkeim in die mindestens eine Kavität der dermalen Matrix eingebracht.

Der Begriff "Haarfollikelkeim" bezeichnet ein frühes Stadium eines Haarfollikels aus dem sich dieser Haarfollikel entwickelt. Vorzugsweise weist der Haarfollikelkeim zumindest die halbe Größe einer physiologischen Hautpapille (DP, Abkürzung für englisch dermal papilla) eines Haarfollikels nach Isolierung derselben auf. Ebenfalls bevorzugt weist der Haarfollikelkeim die ungefähre Form einer physiologischen DP eines Haarfollikels nach Isolierung derselben auf. Die Haarfollikelkeime bestehen aus ca. 2 bis 20000 Zellen, bevorzugt aus 10 bis 15000, besonders bevorzugt aus 1000 bis 10000, ganz besonders bevorzugt aus 1500 bis 5000 Zellen.

Der Haarfollikelkeim umfasst Hautpapillenfibroblasten (DPF, Abkürzung für englisch dermal hair papilla fibroblasts). Weiterhin kann der Haarfollikelkeim Bindegewebsfibroblasten (CTSF, Abkürzung für englisch connective tissue sheath fibroblasts) enthalten. Gegebenenfalls umfasst der Haarfollikelkeim mindestens eine weitere Zellpopulation ausgewählt aus Endothelzellen, Zellen von stromalen vaskulären Fraktionen (SVF), Keratinozyten (KC), Melanozyten (MC) oder Fibroblasten, wie z.B. CTSF.

Die DPF für den Haarfollikelkeim werden aus zumindest einer DP isoliert, wobei die DP aus mindestens einem Haarfollikel stammt.

Methoden für die Gewinnung der DP bzw. DPF sind dem Fachmann bekannt und beispielsweise in den Druckschriften EP 2 274 419 B1 und DE 101 62 814 B4 beschrieben. Die Bereitstellung der DP aus dem Haarfollikel und die Isolierung der DPF aus der DP kann beispielsweise wie folgt durchgeführt werden: Isolierte Haarfollikel werden z.B. mit einer Pinzette am Haarschaft fixiert und die Bindegewebsscheide wird z.B. mit einer weiteren Pinzette diametral vom Haarschaft getrennt, so dass der Bulbus umgestülpt wird und somit die DPF und der Haarschaft mit der Haarmatrix freiliegen. Dadurch können die DP vom restlichen Teil des Haarfollikels getrennt werden was z.B. mithilfe einer Nadel oder Kanüle geschehen kann.

Anschließend werden die isolierten DP beispielsweise in ein Zellkulturgefäß überführt und mechanisch auf der Oberfläche des Zellkulturgefäßes fixiert. Vorzugsweise werden die DPF dadurch erhalten, dass die isolierte DP mechanisch am Boden des Zellkulturgefäßes arretiert wird, z.B. mittels einer Nadelspitze oder einem Skalpell. Die Morphologie der DP bleibt dabei in etwa erhalten, jedoch wird die Basallamina der DP leicht perforiert, so dass die DPF aus der DP migrieren können. Die DPF können auch mithilfe einer enzymatischen Trennung aus der DP erhalten werden.

Es besteht die Möglichkeit, dass die DPF nach ihrer Isolierung und vor ihrer Verwendung im erfindungsgemäßen Verfahren zwischenkultiviert werden, beispielsweise um sie zu vervielfältigen. Entsprechend kann es sich bei den im erfindungsgemäßen Verfahren eingesetzten DPF um Nachkommen oder Klone der ursprünglich isolierten DPF handeln.

Die Haarfollikel, aus denen die DP und die DPF isoliert werden, stammen aus Gewebe. Dem Fachmann sind Verfahren zur Entnahme von Haarfollikeln aus Gewebe bekannt. Bevorzugt werden die Haarfollikel nach Standards guter medizinsicher Praxis aus dem Gewebe entnommen. Vorzugsweise erfolgt die Entnahme der Haarfollikel aus dem Gewebe derart, dass sie im Wesentlichen intakt, d.h. unbeschädigt, bleiben. Die Entnahme der Haarfollikel aus dem Gewebe kann beispielsweise wie folgt durchgeführt werden: i) Abtrennung der Epidermis von der darunterliegenden Dermis bzw. Fettgewebe beim Gewebe, vorzugsweise unter Verwendung eines Skalpells; ii) Präparation von Haaren aus der Dermis bzw. dem Fettgewebe des Gewebes. Die entnommenen Haare umfassen an ihrem proximalen Ende die Haarfollikel. Optional kann vor Schritt ii) die Dermis bzw, das Fettgewebe komprimiert werden, damit die darin vorhandenen Haarfollikel leichter herauspräpariert werden können. Das Komprimieren kann dabei beispielsweise mithilfe einer Pinzette durchgeführt werden. Vorzugsweise findet die Entnahme der Haarfollikel unter dem Mikroskop statt.

Das Gewebe aus dem die Haarfollikel, für z.B. die Isolation der DP bzw. DPF, gewonnen werden können stammt aus einem Säugetier. Dieses Säugetier kann ein Mensch, Affe, Schwein, Hund, Katze oder Nagetier sein, bevorzugt ein Mensch. Das Säugetier stellt also einen Spender für das, den Haarfollikel enthaltene, Gewebe dar. Besonders bevorzugt handelt es sich bei dem Säugetier um einen menschlichen Patienten, der an Haarausfall leidet.

Das Gewebe umfasst bevorzugt Haut. Dabei kann das Gewebe aus jeder behaarten Körperregion gewonnen werden vorzugsweise aus Kopf, Augenbrauen, Bart, Genitalbereich, Bein oder anderen Körperregionen. Vorzugsweise wird das Gewebe mittels einer Biopsie erhalten.

Bevor die Haarfollikel, für z.B. die Isolation der DP bzw. DPF, aus dem Gewebe entnommen werden, kann dieses Gewebe einem oder mehreren Reinigungsschritten unterzogen werden, um möglicherweise vorhandene störende Substanzen von dem Gewebe zu entfernen.

Der Haarfollikelkeim kann neben den DPF noch Bindegewebsfibroblasten (CTSF) enthalten. Die CTSF für den Haarfollikelkeim stammen aus mindestens einem Haarfollikel.

Die Isolierung der CTSF aus dem Haarfollikel kann beispielsweise wie folgt durchgeführt werden: Isolierte Haarfollikel werden z.B. mit einer Pinzette am Haarschaft fixiert und die Bindegewebsscheide wird z.B. mit einer weiteren Pinzette diametral vom Haarschaft getrennt, so dass der Bulbus umgestülpt wird. Dadurch können der proximale Teil des Bulbus mit den CTSF vom restlichen Teil des Haarfollikels getrennt werden was z.B. mithilfe einer Nadel oder Kanüle geschehen kann.

Es besteht die Möglichkeit, dass die CTSF nach ihrer Isolierung und vor ihrer Verwendung im erfindungsgemäßen Verfahren zwischenkultiviert werden, beispielsweise um sie zu vervielfältigen. Entsprechend kann es sich bei den im erfindungsgemäßen Verfahren eingesetzten CTSF um Nachkommen oder Klone der ursprünglich isolierten CTSF handeln.

Die Haarfollikel, aus denen die CTSF isoliert werden, stammen aus Gewebe. Dem Fachmann sind Verfahren zur Entnahme von Haarfollikeln aus Gewebe bekannt. Bevorzugt werden die Haarfollikel nach Standards guter medizinsicher Praxis aus dem Gewebe entnommen. Vorzugsweise erfolgt die Entnahme der Haarfollikel aus dem Gewebe derart, dass sie im Wesentlichen intakt, d.h. unbeschädigt, bleiben. Die Entnahme der Haarfollikel aus dem Gewebe kann beispielsweise wie folgt durchgeführt werden: i) Abtrennung der Epidermis von der darunterliegenden Dermis bzw. Fettgewebe beim Gewebe, vorzugsweise unter Verwendung eines Skalpells; ii) Präparation von Haaren aus der Dermis bzw. dem Fettgewebe des Gewebes. Die entnommenen Haare umfassen an ihrem proximalen Ende die Haarfollikel. Optional kann vor Schritt ii) die Dermis bzw, das Fettgewebe komprimiert werden, damit die darin vorhandenen Haarfollikel leichter herauspräpariert werden können. Das Komprimieren kann dabei beispielsweise mithilfe einer Pinzette durchgeführt werden. Vorzugsweise findet die Entnahme der Haarfollikel unter dem Mikroskop statt.

Das Gewebe aus dem die Haarfollikel, für z.B. die Isolation der CTSF, gewonnen werden können stammt aus einem Säugetier. Dieses Säugetier kann ein Mensch, Affe, Schwein, Hund, Katze oder Nagetier sein, bevorzugt ein Mensch. Das Säugetier stellt also einen Spender für das, den Haarfollikel enthaltene, Gewebe dar. Besonders bevorzugt handelt es sich bei dem Säugetier um einen menschlichen Patienten, der an Haarausfall leidet.

Das Gewebe umfasst bevorzugt Haut. Dabei kann das Gewebe aus jeder behaarten Körperregion gewonnen werden vorzugsweise aus Kopf, Augenbrauen, Bart, Genitalbereich, Bein oder anderen Körperregionen. Vorzugsweise wird das Gewebe mittels einer Biopsie erhalten.

Bevor die Haarfollikel, für z.B. die Isolation der CTSF, aus dem Gewebe entnommen werden, kann dieses Gewebe einem oder mehreren Reinigungsschritten unterzogen werden, um möglicherweise vorhandene störende Substanzen von dem Gewebe zu entfernen.

Die DPF und CTSF werden also aus isolierten Haarfollikeln gewonnen. Dabei können die DPF und CTSF aus demselben Haarfollikel gewonnen werden. Die DPF und CTSF können aber auch aus unterschiedlichen Haarfollikeln gewonnen werden. Werden die DPF und CTSF aus unterschiedlichen Haarfollikeln gewonnen, können diese Haarfollikel aus dem gleichen Gewebe isoliert worden sein, beispielsweise aus einem Gewebe, welches z.B. aus dem Nackenbereich des Kopfes eines Spenders entnommen wurde. Werden die DPF und CTSF aus unterschiedlichen Haarfollikeln gewonnen, so können diese Haarfollikel auch aus verschiedenen Geweben isoliert worden sein, so dass beispielsweise eins der Gewebe z.B. aus dem Nackenbereich des Kopfes eines Spenders entnommen wurde und das andere Gewebe z.B. aus dem Bart eines Spenders. Im letzteren Fall kann es sich bei dem Spender um denselben Spender oder einen anderen Spender handeln. Sofern es sich um einen anderen Spender handelt, ist dieser vorzugsweise ein allogener Spender. Vorzugsweise handelt es sich um denselben, also autologen, Spender.

Der Haarfollikelkeim kann neben den DPF und gegebenenfalls den CTSF noch mindestens eine weitere Zellpopulation ausgewählt aus Endothelzellen, Zellen von stromalen vaskulären Fraktionen (SVF), Keratinozyten (KC), Melanozyten (MC) oder Fibroblasten enthalten. Die gegebenenfalls im Haarfollikelkeim vorhandenen Endothelzellen (EZ) können mithilfe von im Stand der Technik bekannten Methoden zur Gewinnung von EZ isoliert werden.

Die EZ können aus Lymph-oder Blutgefäßen gewonnen werden, bevorzugt aus Lymph-oder Blutgefäßen, welche sich in der Nähe von Haarfollikeln befinden. Vorzugsweise werden die EZ aus einem humanen Spender gewonnen. Besonders bevorzugt handelt es sich dabei um autologe EZ. Es besteht die Möglichkeit, dass die EZ nach ihrer Isolierung und vor ihrer Verwendung im erfindungsgemäßen Verfahren zwischenkultiviert werden, beispielsweise um sie zu vervielfältigen. Entsprechend kann es sich bei den im erfindungsgemäßen Verfahren eingesetzten EZ um Nachkommen oder Klone der ursprünglich isolierten EZ handeln.

Der Haarfollikelkeim kann Zellen von stromalen vaskulären Fraktionen (SVF) umfassen. Die SVF umfassen verschiedene Zellpopulationen wie Vorläufer von Fettzellen (Präadipozyten), Endothelzellen, endotheliale Muskelzellen, Fibroblasten, Makrophagen oder Blutzellen. Die SVF können beispielsweise mittels Fettgewebsexzision oder Liposuktion gewonnen werden. Methoden zur Gewinnung der SVF sind dem Fachmann bekannt. So ist die Isolierung von SVF aus Fettgewebe beispielsweise in der Druckschrift WO 2014 036 094 A1 beschrieben. Vorzugsweise wird die SVF aus einem humanen Spender gewonnen, besonders bevorzugt handelt es sich dabei um eine autologe SVF. Es besteht die Möglichkeit, dass bestimmte Bestandteile der SVF nach ihrer Gewinnung aus dem Spender aus der SVF entfernt werden, was durch einen oder mehrere Wasch-, Reinigungs- oder Isolierungsschritte erfolgen kann oder auch durch gezieltes Abtrennen oder Entfernen der Bestandteile. Ebenfalls ist es möglich, dass die SVF nach ihrer Isolierung und vor ihrer Verwendung im erfindungsgemäßen Verfahren zwischenkultiviert werden, beispielsweise um sie zu vervielfältigen. Entsprechend kann es sich bei den im erfindungsgemäßen Verfahren eingesetzten SVF um Nachkommen oder Klone der ursprünglich isolierten SVF handeln. Durch die Anwesenheit der SVF, wird eine erhöhte bzw. schnellere Zellmultiplikation der DPF und CTSF erreicht und entsprechend die Bildung der Haarfollikelkeime beschleunigt.

Weiterhin kann der Haarfollikelkeim Keratinozyten (KC), Melanozyten (MC) und/oder CTSF umfassen. Die KC, MC und/oder CTSF müssen dabei nicht von einem Haarfollikel stammen, sondern können aus anderen Säugetier-Geweben, wie z.B. Haut, gewonnen werden. Methoden zur Gewinnung der KC, MC und CTSF sind dem Fachmann bekannt und sind beispielsweise in der Druckschrift DE 101 62 814 B4 sowie in Toma et al., Stem cells 23, 727-37 (2005), Barrandon and Green, Proc. Natl. Acad. Sci. 82, 5390-4 (1985), Tobin et al., J. Invest. Dermatology 104(1), 86-88 (1995) und Magerl et al., Methods Exp. Dermat. 11, 381-385 (2002) beschrieben. Vorzugsweise kann die mindestens eine weitere Zellpopulation aus einem Haarfollikel gewonnen werden und/oder wird sie aus einem Haarfollikel gewonnen.

Dabei können die KC, MC und/oder CTSF aus demselben Haarfollikel gewonnen werden. Die KC, MC und/oder CTSF können aber auch aus unterschiedlichen Haarfollikeln gewonnen werden. Werden die KC, MC und/oder CTSF aus unterschiedlichen Haarfollikeln gewonnen, können diese Haarfollikel aus dem gleichen Gewebe isoliert worden sein, beispielsweise aus einem Gewebe, welches z.B. aus dem Nackenbereich des Kopfes eines Spenders entnommen wurde. Werden die KC, MC und/oder CTSF aus unterschiedlichen Haarfollikeln gewonnen, so können diese Haarfollikel auch aus verschiedenen Geweben isoliert worden sein, so dass beispielsweise eins der Gewebe z.B. aus dem Nackenbereich des Kopfes eines Spenders entnommen wurde und ein anderes Gewebe z.B. aus dem Bart eines Spenders. Im letzteren Fall kann es sich bei dem Spender um denselben Spender oder einen anderen Spender handeln. Sofern es sich um einen anderen Spender handelt, ist dieser vorzugsweise ein allogener Spender. Vorzugsweise handelt es sich um denselben, also autologen, Spender.

Besonders bevorzugt wird die mindestens eine weitere Zellpopulation aus demselben Haarfollikel gewonnen, aus dem die DP isoliert wurde. Es besteht die Möglichkeit, dass die KC, MC und/oder CTSF, nach ihrer Isolierung und vor ihrer Verwendung im erfindungsgemäßen Verfahren zur Herstellung der Haarfollikel zwischenkultiviert werden, beispielsweise um sie zu vervielfältigen. Entsprechend kann es sich bei den im erfindungsgemäßen Verfahren eingesetzten KC, MC und/oder CTSF um Nachkommen oder Klone der ursprünglich isolierten KC, MC und/oder CTSF handeln.

Vorzugsweise umfasst der Haarfollikelkeim DPF und CTSF. Besonders bevorzugt umfasst der Haarfollikelkeim DPF, CTSF und SVF. Ganz besonders bevorzugt umfasst der Haarfollikelkeim DPF, CTSF, SVF, KC und MC.

Der Haarfollikelkeim kann eine Beschichtung umfassen, welche ein oder mehrere unterschiedliche extrazelluläre Matrixproteine wie beispielsweise Kollagen IV, Fibronektin und/oder Laminin enthält. Diese Beschichtung kann durch die DPF oder CTSF selbst entstehen, während diese den Haarfollikelkeim ausbilden. Alternativ oder zusätzlich dazu kann der Haarfollikelkeim auch nach seiner Formierung mit dem einen oder mehreren unterschiedlichen extrazellulären Matrixproteinen beschichtet werden.

Wird in Schritt b) des erfindungsgemäßen Verfahrens ein Haarfollikelkeim in die dermale Matrix eingebracht, so kann dieser Schritt b) beispielsweise wie folgt durchgeführt werden: Vorzugsweise werden die Kavitäten der dermalen Matrix zunächst mit extrazellulären Matrixproteinen beschichtet und anschließend mit DPF und/oder CTSF besiedelt. Bevorzugt werden zunächst die CTSF in die Kavitäten eingebracht und anschließend die DPF. Danach können optional EZ und/oder SVF in die Kavitäten eingebracht werden. In einem nächsten Schritt werden MC und/oder KC hinzugefügt, vorzugsweise MC und KC.

Durch das Einbringen des mindestens einen Haarfollikels und/oder Haarfollikelkeims in die dermale Matrix entsteht ein Hautsurrogat. Erfindungsgemäß wird unter "Hautsurrogat" ein nicht vollwertiger Hautersatz verstanden. Das Hautsurrogat wird als Zwischenprodukt verstanden, aus dem das Hautäquivalent hergestellt wird.

Vorzugsweise erfolgt nach Schritt b) eine Inkubation des Hautsurrogats von mindestens 10min, bevor Schritt c) durchgeführt wird. Bevorzugt erfolgt die Inkubation für 20min bis 48h, besonders bevorzugt für 30min bis 24h, besonders bevorzugt für 1h bis 12h.

Im nachfolgenden Schritt c) des erfindungsgemäßen Verfahrens wird mindestens eine weiteren Zellpopulation ausgewählt aus Fibroblasten, Endothelzellen, Adipozyten, Nervenzellen, Drüsenzellen, Melanozyten oder Keratinozyten zum Hautsurrogat hinzugefügt, um das Hautäquivalent herzustellen. Methoden zur Gewinnung der Adipozyten, Nervenzellen, Drüsenzellen sind dem Fachmann bekannt.

Vorzugsweise findet nach Schritt c) des erfindungsgemäßen Verfahrens eine Inkubation des Hautäquivalents von mindestens 10 min statt, bevor das Hautäquivalent, beispielsweise als Transplantat oder für die Testung von Substanzen, weiterverwendet wird. Bevorzugt erfolgt die Inkubation für mindestens 20 min, besonders bevorzugt für 1h bis 21 Tage, besonders bevorzugt für 5 bis 15 Tage, ganz besonders bevorzugt für 8 bis 12 Tage.

Die vorliegende Erfindung bezieht sich auch auf ein Hautäquivalent, welches durch das erfindungsgemäße Verfahren hergestellt wird.

Die vorliegende Erfindung umfasst außerdem ein Transplantat, welches eine effektive Menge des Hautäquivalents optional zusammen mit pharmazeutisch tolerierbaren Adjuvantien umfasst.

In einer Ausführungsform umfasst das Verfahren zur Herstellung des Hautäquivalents die Schritte: a) Herstellen einer dermalen Matrix enthaltend eine erste Schicht und eine zweite Schicht, umfassend die Schritte: i) Bereitstellen der ersten Schicht; ii) Bereitstellen einer polymerisierbaren Lösung umfassend eine Flüssigkeit und mindestens ein Polymer ausgewählt aus Kollagen, Elastin und/oder Hyaluron; iii) Hinzufügen der polymerisierbaren Lösung zur ersten Schicht; iv) Polymerisierung der Lösung zur zweiten Schicht; v) Komprimieren der zweiten Schicht, wobei durch das Komprimieren der Flüssigkeitsgehalt der zweiten Schicht reduziert wird und die erste Schicht und die zweite Schicht zur dermalen Matrix zusammengefügt werden; und vi) Einbringen von mindestens einer Kavität in die dermale Matrix; b) Einbringen von mindestens einem Haarfollikel und/oder Haarfollikelkeim in die mindestens eine Kavität der in Schritt a) hergestellten dermalen Matrix zur Herstellung eines Hautsurrogats; und c) Hinzufügen von mindestens einer weiteren Zellpopulation ausgewählt aus Fibroblasten, Endothelzellen, Adipozyten, Nervenzellen, Drüsenzellen, Melanozyten oder Keratinozyten zum Hautsurrogat zur Herstellung des Hautäquivalents. Vorzugsweise handelt es sich bei der ersten Schicht um eine künstliche Hautsubstanz, besonders bevorzugt umfasst die künstliche Hautsubstanz Kollagen und/oder Elastin, insbesondere bevorzugt Kollagen und Elastin.

Die polymerisierbare Lösung umfasst als Polymer Kollagen, vorzugsweise Kollagen des Typs I. Besonders bevorzugt umfasst die polymerisierbare Lösung zusätzlich Bindegewebszellen, vorzugsweise Fibroblasten.

Vorzugsweise wird vor Schritt iv) das Polymer in der polymerisierbaren Lösung durch Bewegung strukturiert.

Bevorzugt erfolgen die Polymerisierung der Lösung zur zweiten Schicht in Schritt iv) und das Einbringen von mindestens einer Kavität in die dermale Matrix in Schritt vi) gleichzeitig. Besonders bevorzugt erfolgen Schritt iv) und Schritt vi) gleichzeitig und unter Bewegung, besonders bevorzugt unter Rotationsbewegung.

Das Komprimieren der zweiten Schicht in Schritt v) erfolgt vorzugsweise durch Zentrifugieren.

Vorzugsweise werden in Schritt b) mindestens ein Haarfollikelkeim in die mindestens eine Kavität der dermalen Matrix eingebracht, wobei die Kavität besonders bevorzugt mit extrazellulären Matrixproteinen beschichtet wird, bevor zunächst CTSF und dann DPF in die Kavität eingebracht werden. Vorzugsweise werden zusätzlich noch EZ und/ oder SVF in die Kavität eingebracht. Anschließend werden MC und/oder KC, vorzugsweise MC und KC in die Kavitäten hinzugefügt.

In Schritt c) werden zu dem in Schritt b) generierten Hautsurrogat vorzugsweise EZ, Adipozyten, MC und/oder KC hinzugefügt, vorzugsweise EZ, Adipozyten, MC und KC. Besonders bevorzugt werden zusätzlich noch Nervenzellen und Drüsenzellen zum Hautsurrogat hinzugefügt.

Die vorliegende Erfindung bezieht sich auch auf die Verwendung des Hautäquivalents, welches nach dem erfindungsgemäßen Verfahren hergestellt wurde, und/oder des Transplantats umfassend das Hautäquivalent für die therapeutische Hautwiederherstellung oder die Behandlung reduzierter Hautmenge. Hierbei ist es bevorzugt, dass für die Herstellung des Hautäquivalents allogene Zellen verwendet werden, was bedeutet dass der Spender der verwendeten Zellen und der Empfänger des hergestellten Hautäquivalents der gleichen Art angehören, d.h. dass sowohl Spender als auch Empfänger beispielsweise Menschen sind. Besonders bevorzugt ist es für die therapeutische Behandlung eines Zustandes von reduzierter Hautmenge, dass für die Herstellung des Hautäquivalents autologe Zellen verwendet werden, was bedeutet dass der Spender der verwendeten Zellen und der Empfänger des hergestellten Hautäquivalents identisch ist, beispielsweise also derselbe Mensch ist.

Des Weiteren bezieht sich die vorliegende Erfindung auch auf die Verwendung des erfindungsgemäßen Hautäquivalents zur in vitro Testung von Substanzen auf kosmetische, pharmakologische oder toxische Eigenschaften.

Ferner bezieht sich die vorliegende Erfindung auf die Verwendung des erfindungsgemäßen Hautäquivalents zur in vitro Testung von haut- und/oder haar-regulierenden Substanzen.

Die vorliegende Erfindung umfasst auch ein Kit zur Durchführung des erfindungsgemäßen Verfahrens. Dazu kann das Kit das erfindungsgemäße Hautäquivalent und/oder das Transplantat umfassen, um beispielsweise die erfinderischen Verfahren zur therapeutischen Hautwiederherstellung oder der Behandlung eines Zustandes von reduzierter Hautmenge oder dem Screening von Substanzen durchzuführen. Gegebenenfalls kann das Kit auch eine Gebrauchsanweisung zur Verwendung des Kits und/oder zur Durchführung des erfindungsgemäßen Verfahrens enthalten. Ferner kann das Kit weitere Bestandteile zur Durchführung des erfindungsgemäßen Verfahrens enthalten wie beispielsweise Reaktionsgefäße, Filter, Lösungen und/oder andere Mittel.

Die vorliegende Erfindung bezieht sich auch auf ein Verfahren zum in vitro Screening von Substanzen mit haut- und/oder haar-regulierenden Eigenschaften, welches die folgenden Schritte umfasst: a) Bereitstellen einer Probe des Hautäquivalents; b) Aufteilen der entsprechenden Probe in Portionen; c) inkubieren mindestens einer Portion mit Substanzen, die getestet werden sollen; und d) Vergleichen der Parameter der Haut- und/oder Haareigenschaften in der Portion mit einer anderen Portion, die nicht mit den Substanzen inkubiert wurde.

Die vorliegende Erfindung umfasst auch ein Testsystem umfassend das nach dem erfindungsgemäßen Verfahren hergestellte Hautäquivalent zur in vitro Untersuchung der Ursachen von krankheitsbedingtem Haarausfall.

Die Erfindung wird nachfolgend anhand von Ausführungsbeispielen und den dazugehörigen Figuren näher erläutert.

### Figuren

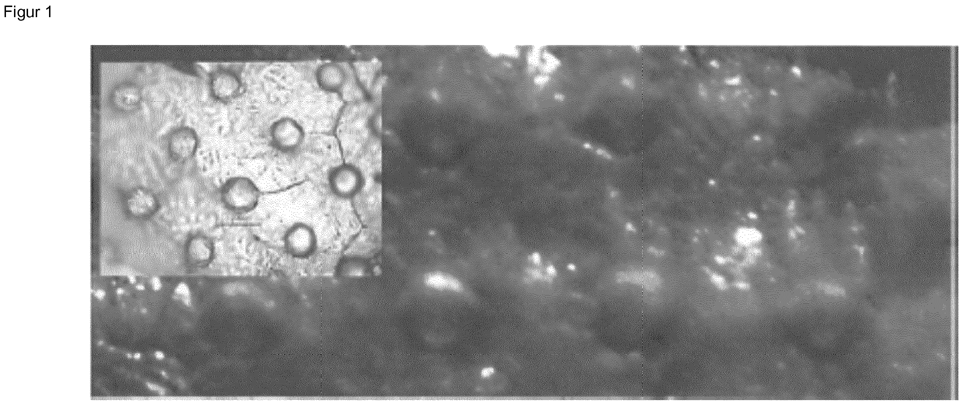

- Figur 1: Fotografische Abbildung einer dermalen Matrix.

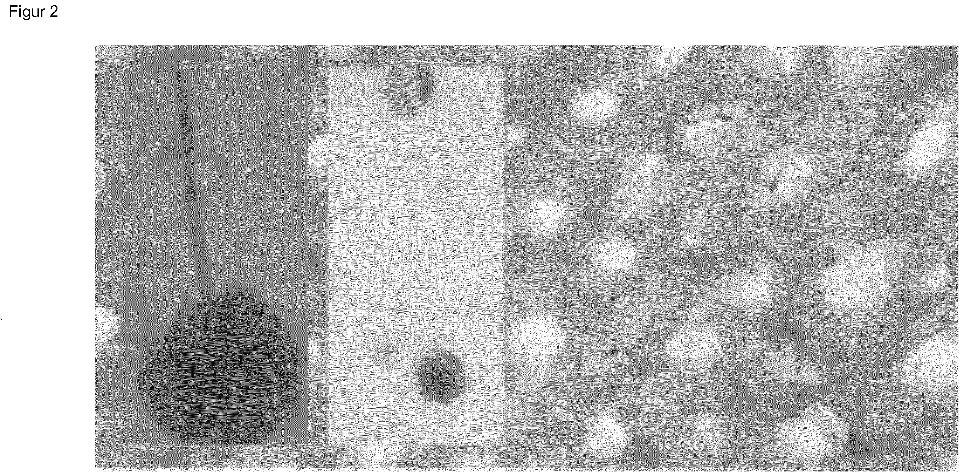

- Figur 2: Fotografische Abbildung eines Hautäquivalents, eines Haarfollikels und Haarfollikelkeimen.

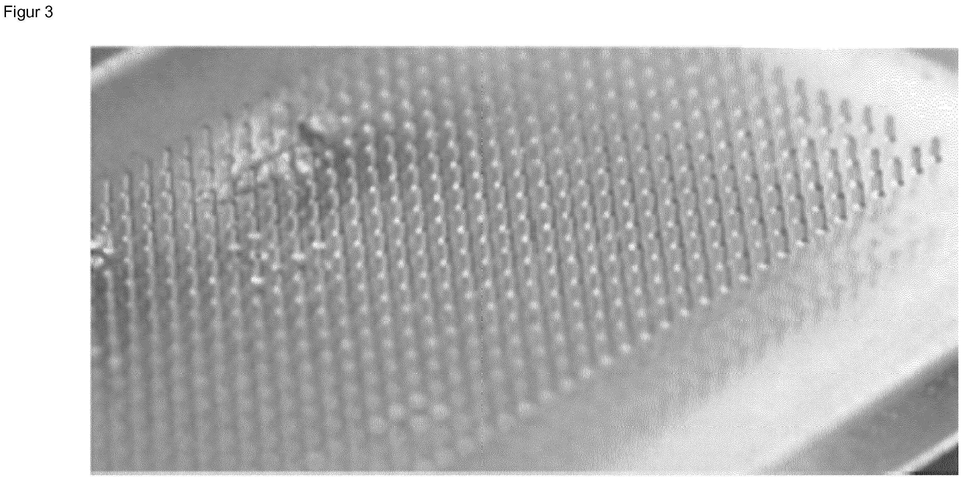

- Figur 3: Fotografische Abbildung einer Abdruckform zur Einbringung von Kavitäten in eine dermale Matrix.

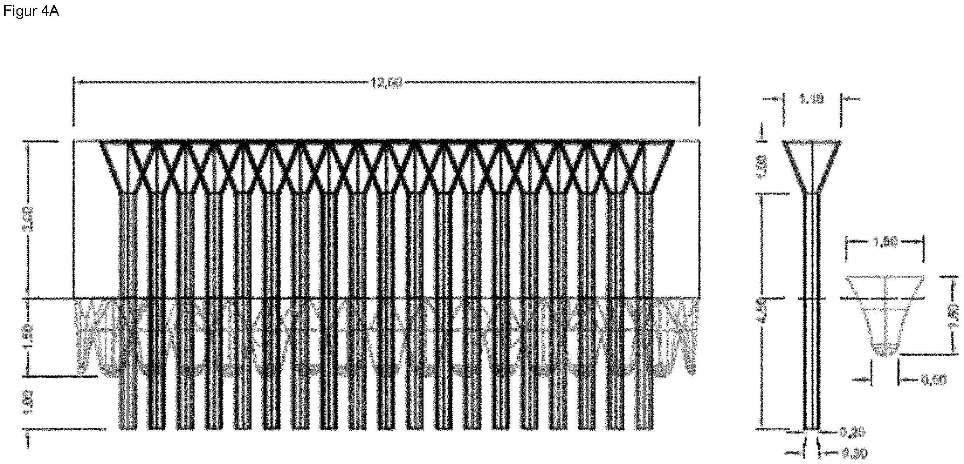

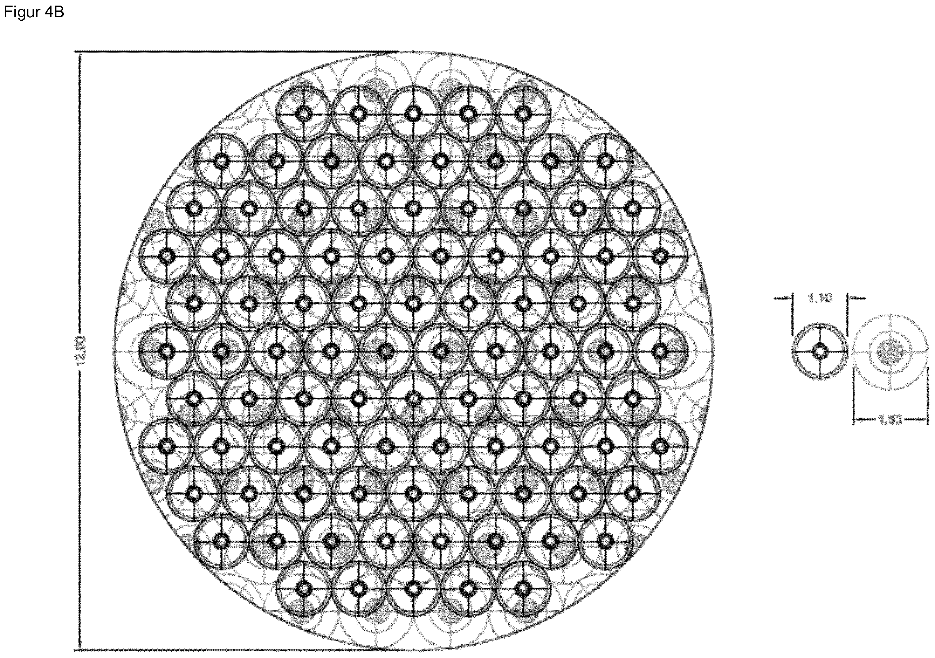

- Figur 4: Schematische Zeichnung einer Abdruckform zur Einbringung von Kavitäten in eine dermale Matrix. A: Schnittansicht, B: Draufsicht.

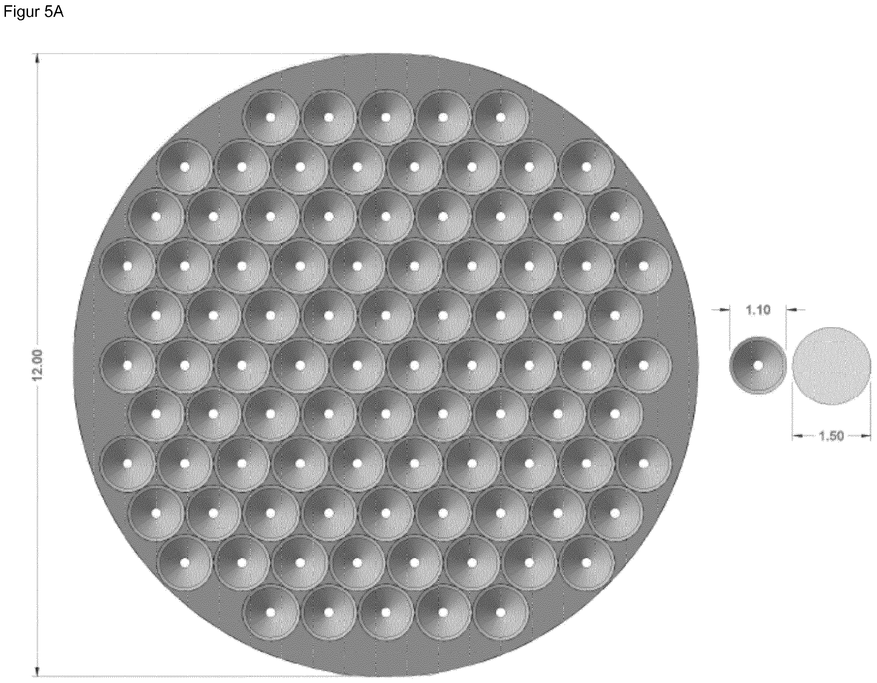

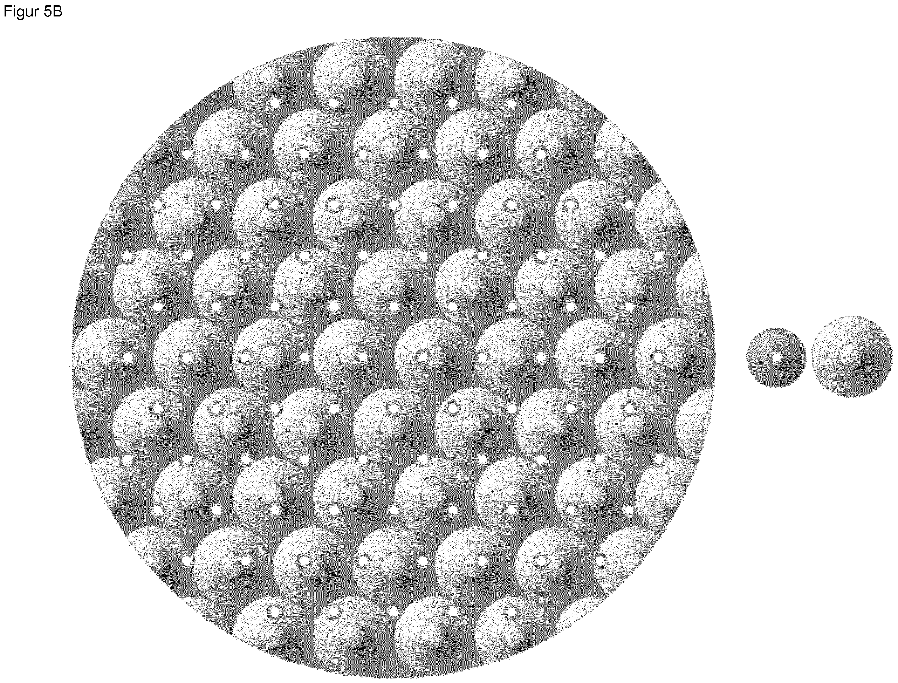

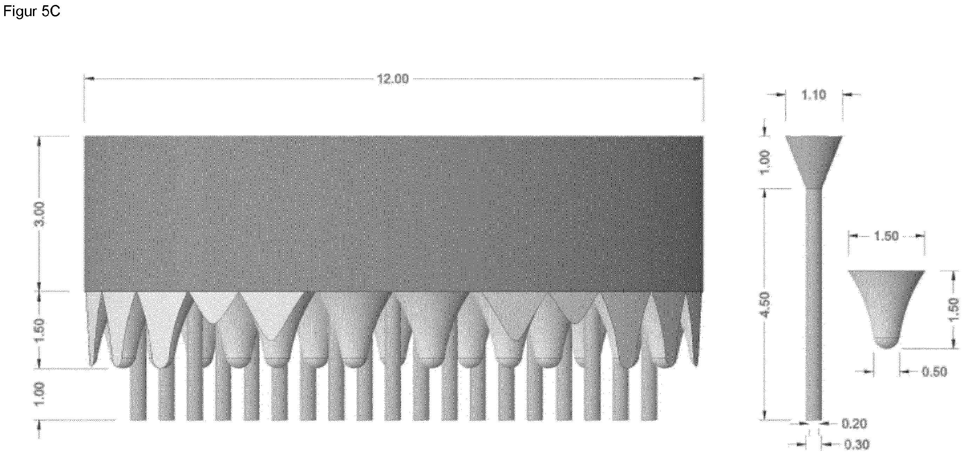

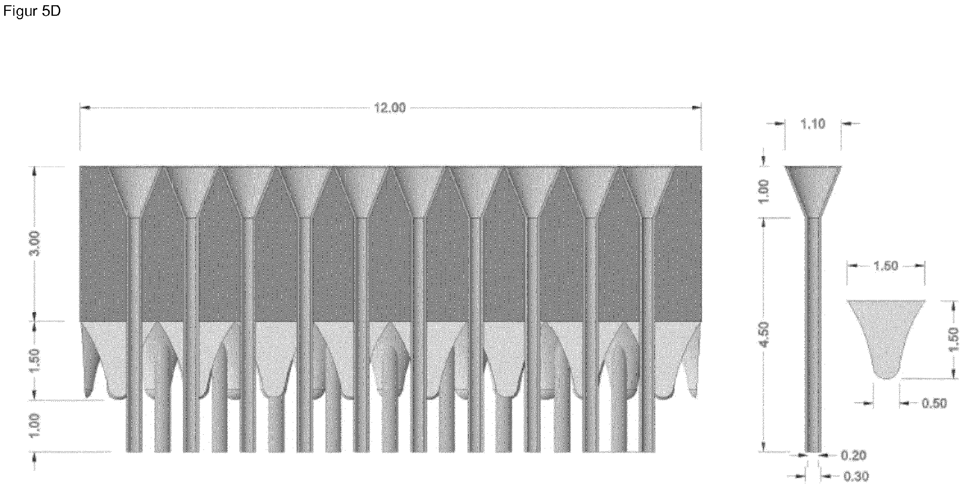

- Figur 5: Schematische Zeichnung einer Abdruckform zur Einbringung von Kavitäten in eine dermale Matrix. A: Draufsicht auf Oberseite der Abdruckform, B: Draufsicht auf die Unterseite der Abdruckform, C und D: Seitenansicht.

### Beispiele

### Isolation der verschiedenen Zelltypen aus Haarfollikeln

Die Isolierung von DPF, CTSF, MC, KC aus dem humanen Haarfollikel erfolgt in modifizierter Form der Standardprotokolle (z.B. beschrieben in "Magerl et al."). Isolierte Haarfollikel aus Stanzbiopsien oder präparierte Haarfollikel aus einer FUT Haartransplantationen werden mit einer Pinzette am Haarschaft fixiert und die Bindegewebsscheide angeschnitten und diametral mit einer weiteren Pinzette vorsichtig voneinander getrennt, so dass der Bulbus umgestülpt wird und Dermale Papille und der Haarschaft mit der Haarmatrix freiliegen. Dadurch können der proximale Teil des Bulbus mit den Bindegewebsscheiden-Fibroblasten und die Dermalen Papille sehr leicht mit Hilfe einer Nadel/Kanüle vom Rest des Haarfollikels getrennt werden. Auch der Haarschaft, der die ebenso benötigten Haarmatrix-Keratinozyten und Melanozyten beinhaltet wird dabei für die weitere Kultivierung optimal präpariert.

Die so extrahierten Dermalen Papillen und Bindegewebshüllen der entnommenen Haarfollikel werden jeweils in einem separaten Gefäß mit Medium gesammelt. Mittels schonendem Gewebeaufschluss durch einen Gewebe-Dissoziierer und dem dazugehörigen Extractions-Kit (z.B. gentleMACS Dissociator # 130-093-235, whole skin dissociation kit #130-101-540, Miltenyi Biotec) werden die DPF und CTSF aus dem Gewebeverband herausgelöst und vereinzelt. Hierzu werden jeweils die isolierten Gewebefragmente

(Dermale Papille, Bindegewebshülle und Haarschaft mit epithelialen / neuroektodermalen Zellen), 435 µl Puffer L, und ein Enzym-Mix aus 12,5 mg Enzym P und/oder 4.50 mg Enzym D und/oder 2,5 mg Enzym A in ein gentleMACS C-Tube gegeben und vorsichtig gemischt.

Die Probe wird in einem Wasserbad bei 37 ° C für 1-3 Stunden oder über Nacht Inkubiert, wobei längere Inkubationszeiten die Zellausbeute erhöhen. Nach der Inkubation wird die Probe durch Zugabe von 0,5 ml kaltem Zellkulturmedium verdünnt. Das C-Tube wird verschlossen und kopfstehend auf die Hülse des gentleMACS Dissociators befestigen. Anschließend wird das das Programm "h_skin_01" durchlaufen. Nach Beendigung des Programms, wird ein kurzer Zentrifugationsschritt angeschlossen, um das Probenmaterial am Boden zu sammeln. Die Zellen können mit frischem Medium gewaschen werden und die Zellsuspension durch 70 µm -Filter separiert werden. Auf eine oben beschriebene Hinzugabe von Enzymen, die bei einer direkten autologen Therapie unerwünscht sind, kann mit Hilfe weiterer Dissoziationsläufe verzichtet werden obwohl dann mit geringeren Zellausbeuten gerechnet werden muss.

### Herstellung von Zellen einer stromalen vaskulären Fraktion (SVF)

Eine 1 Liter Fraktion einer tumeszenten Fettabsaugung aus subkutanen Bauch- oder Hüftfett, wird entnommen und z.B mit dem etablierten Verfahren PureGraft™ (Cytori GmbH, Schweiz) aufbereitet. Dabei wird zentrifugiert und konzentriert um die tumeszente Lösung zu entfernen. Anschließend wird für 60min bei 37°C in 0.15% (W/V) Collagenase NB 6 GMP Grade aus Clostridium histolyticum (0.12U/mg collagenase; SERVA Electrophoresis GmbH) verdünnt in Phosphat-gepufferter Saline (PBS; Gibco) verdaut. Nach der Zentrifugation bei 180 g für 10 Minuten, wird die lipidreiche Schicht verworfen und das Zellpellet einmal mit PBS gewaschen. Erytrozythen werden durch 2minütige Inkubation in Lysispuffer (0.15M Ammoniumchloride,Sigma-Aldrich) aufgelöst. Die gewonnene stromale vaskuläre Fraktion (SVF) wird in Komplettmedium (KM, Gibco) aufgenommen.

### Herstellung eines Hautäquivalents

Das Dermisäquivalent wird auf der Basis einer 2mm starken Kollagen/Elastin Matrix (Matriderm®, Dr. Suwelack Skin & Health Care AG) aufgebaut. Mittels einer Rundstanze wird ein Stück ausgestanzt und in ein Transwell® überführt. Dieses wird auf Eis mit einer Kollagen-I-Lösung, die dermale Fibroblasten enthält, beschichtet. Der Silikonstempel mit den Zylindern wird leicht rotiert. Dann wird das Gel für15 min polymerisiert und anschließend in eine Zentrifuge überführt. Nach der Zentrifugation wird das Wasser entnommen und die gebildeten Kavitäten nacheinander mit ECM beschichtet und den Haarfollikelzellen besiedelt.

## Patentansprüche

1. Verfahren zur Herstellung eines Hautäquivalents, umfassend die Schritte:
a) Herstellen einer dermalen Matrix enthaltend eine erste Schicht und eine zweite Schicht, umfassend die Schritte:
i) Bereitstellen der ersten Schicht;
ii) Bereitstellen einer polymerisierbaren Lösung umfassend eine Flüssigkeit und mindestens ein Polymer ausgewählt aus Kollagen, Elastin und/oder Hyaluron;
iii) Hinzufügen der polymerisierbaren Lösung zur ersten Schicht;
iv) Polymerisierung der Lösung zur zweiten Schicht;
v) Komprimieren der zweiten Schicht, wobei durch das Komprimieren der Flüssigkeitsgehalt der zweiten Schicht reduziert wird und die erste Schicht und die zweite Schicht zur dermalen Matrix zusammengefügt werden; und
vi) Einbringen von mindestens einer Kavität in die dermale Matrix;
b) Einbringen von mindestens einem Haarfollikel und/oder Haarfollikelkeim in die mindestens eine Kavität der in Schritt a) hergestellten dermalen Matrix zur Herstellung eines Hautsurrogats; und
c) Hinzufügen von mindestens einer weiteren Zellpopulation ausgewählt aus Fibroblasten, Endothelzellen, Adipozyten, Nervenzellen, Drüsenzellen, Melanozyten oder Keratinozyten zum Hautsurrogat zur Herstellung des Hautäquivalents.

2. Verfahren nach Anspruch 1, wobei die erste Schicht eine natürliche Hautsubstanz oder eine künstliche Hautsubstanz umfasst.

3. Verfahren nach Anspruch 1 oder 2, wobei die erste Schicht keine lebenden Zellen enthält.

4. Verfahren nach einem der vorhergehenden Ansprüche, wobei die polymerisierbare Lösung zusätzlich Zellen enthält.

5. Verfahren nach einem der vorhergehenden Ansprüche, wobei vor Schritt iv) das Polymer in der polymerisierbaren Lösung durch Bewegung strukturiert wird.

6. Verfahren nach einem der vorhergehenden Ansprüche, wobei das Komprimieren der zweiten Schicht durch Zentrifugieren, Sedimentation oder mechanische Krafteinwirkung erfolgt.

7. Verfahren nach einem der vorhergehenden Ansprüche, wobei die Verfahrensschritte iv), v) oder vi) nacheinander und/oder gleichzeitig stattfinden.

8. Verfahren nach einem der vorhergehenden Ansprüche, wobei das Einbringen der mindestens einen Kavität in die dermale Matrix in Schritt vi) während oder nach der Polymerisierung in Schritt iv) erfolgt, vorzugsweise während der Polymerisierung.

9. Verfahren nach einem der vorhergehenden Ansprüche, wobei die mindestens eine Kavität durch Einstechen, Ausschneiden oder mittels einer Abdruckform in die dermale Matrix eingebracht wird.

10. Verfahren nach einem der vorhergehenden Ansprüche, wobei die Kavitäten einen mittleren Durchmesser von ≥0,001 mm bis 5mm aufweisen, bevorzugt von 0,005mm bis 1mm, besonders bevorzugt von 0,01mm bis 0,08mm, ganz besonders bevorzugt von 0,02mm bis 0,06mm, insbesondere bevorzugt von 0,05mm bis 0,04mm.

11. Verfahren nach einem der vorhergehenden Ansprüche, wobei die Kavitäten eine Tiefe von ≥0,05mm bis 10mm aufweisen, bevorzugt von 0,1mm bis 8mm, besonders bevorzugt von 0,15mm bis 6mm, ganz besonders bevorzugt von 0,2mm bis 4mm, insbesondere bevorzugt von 0,5mm bis 2,5mm.

12. Verfahren nach einem der vorhergehenden Ansprüche, wobei eine, mehrere oder alle Kavitäten der dermalen Matrix vor dem Einbringen des Haarfollikels und/oder Haarfollikelkeims in Schritt b) mit extrazellulären Matrixproteinen beschichtet wird.

13. Hautäquivalent hergestellt durch das Verfahren nach einem der Ansprüche 1 bis 12.

14. Transplantat umfassend eine effektive Menge an Hautäquivalent nach Anspruch 13, optional zusammen mit pharmazeutisch tolerierbaren Adjuvantien.

15. Verfahren zum in vitro Screening von Substanzen mit haut- und/oder haar-regulierenden Eigenschaften, umfassend die Schritte:
a) Bereitstellen einer Probe des Hautäquivalents nach Anspruch 13;
b) Aufteilen der Probe in Portionen;
c) inkubieren mindestens einer Portion mit Substanzen, die getestet werden sollen; und
d) Vergleichen der Parameter der Haut- und/oder Haareigenschaften in der Portion mit einer anderen Portion, die nicht mit den Substanzen inkubiert wurde.

## Claims

1. A method for producing a skin equivalent, comprising the steps of:
a) producing a dermal matrix including a first layer and a second layer, comprising the steps of:
i) providing the first layer;
ii) providing a polymerizable solution comprising a liquid and at least one polymer selected from collagen, elastin and/or hyaluronic acid;
iii) adding the polymerizable solution to the first layer;
iv) polymerizing the solution to the second layer;
v) compressing the second layer, wherein, through the compression, the liquid content of the second layer is reduced and the first layer and the second layer are joined to form the dermal matrix; and
vi) introducing at least one cavity into the dermal matrix;
b) introducing at least one hair follicle and/or hair follicle germ into the at least one cavity of the dermal matrix produced in step a) to produce a skin surrogate; and
c) adding at least one further cell population, selected from fibroblasts, endothelial cells, adipocytes, nerve cells, gland cells, melanocytes or keratinocytes, to the skin surrogate to produce the skin equivalent.

2. The method according to Claim 1, wherein the first layer comprises a natural skin substance or an artificial skin substance.

3. The method according to Claim 1 or 2, wherein the first layer does not contain living cells.

4. The method according to any one of the preceding claims, wherein the polymerizable solution additionally includes cells.

5. The method according to any one of the preceding claims, wherein, before step iv), the polymer in the polymerizable solution is structured by movement.

6. The method according to any one of the preceding claims, wherein the compressing of the second layer is carried out by centrifuging, sedimentation or mechanical force.

7. The method according to any one of the preceding claims, wherein the method steps iv), v) or vi) take place successively and/or simultaneously.

8. The method according to any one of the preceding claims, wherein the introducing of the at least one cavity into the dermal matrix in step vi) is carried out during or after the polymerization in step iv), preferably during the polymerization.

9. The method according to any one of the preceding claims, wherein the at least one cavity is introduced into the dermal matrix through punching, cutting out or by means of an impression mold.

10. The method according to any one of the preceding claims, wherein the cavities have a mean diameter of ≥0.001 mm to 5 mm, preferably of 0.005 mm to 1 mm, particularly preferably of 0.01 mm to 0.08 mm, very particularly preferably of 0.02 mm to 0.06 mm, in particular preferably of 0.05 mm to 0.04 mm.

11. The method according to any one of the preceding claims, wherein the cavities have a depth of ≥0.05 mm to 10 mm, preferably of 0.1 mm to 8 mm, particularly preferably of 0.15 mm to 6 mm, very particularly preferably of 0.2 mm to 4 mm, in particular preferably of 0.5 mm to 2.5 mm.

12. The method according to any one of the preceding claims, wherein one, several or all cavities of the dermal matrix are coated with extracellular matrix proteins before the introducing of the hair follicle and/or hair follicle germ in step b).

13. A skin equivalent produced by the method according to any one the claims 1 to 12.

14. A transplant comprising an effective quantity of skin equivalent according to Claim 13, optionally together with pharmaceutically tolerable adjuvants.

15. A method for in vitro screening of substances with skin- and/or hair-regulating characteristics, comprising the steps of:
a) providing a sample of the skin equivalent according to Claim 13;
b) dividing the sample into portions;
c) incubating at least one portion with substances to be tested; and
d) comparing the parameters of the skin and/or hair characteristics in the portion with another portion which was not incubated with the substances.

## Revendications

1. Procédé pour la fabrication d'un équivalent de peau, comprenant les étapes de :
a) fabrication d'une matrice dermale comprenant une première couche et une deuxième couche, comprenant les étapes de :
i) mise à disposition de la première couche ;
ii) mise à disposition d'une solution polymérisable comprenant un liquide et au moins un polymère sélectionné parmi le groupe se composant de collagène, d'élastine et/ou d'hyaluron ;
iii) ajout de la solution polymérisable à la première couche ;
iv) polymérisation de la solution en deuxième couche ;
v) compression de la deuxième couche, la teneur en liquide de la deuxième couche étant réduite par la compression, et la première couche et la deuxième couche étant ajoutées à la matrice dermale ; et
vi) réalisation d'au moins une cavité dans la matrice dermale ;
b) apport d'au moins un follicule pileux et/ou d'un germe de follicule pileux dans l'au moins une cavité de la matrice dermale fabriquée à l'étape a) pour la fabrication d'un substitut cutané ; et
c) ajout d'au moins une autre population de cellules sélectionnées parmi le groupe se composant de fibroblastes, cellules endothéliales, adipocytes, neurones, cellules glandulaires, mélanocytes ou kératinocytes pour le substitut cutané pour la fabrication de l'équivalent de peau.

2. Procédé selon la revendication 1, la première couche comprenant une substance cutanée naturelle ou une substance cutanée artificielle.

3. Procédé selon la revendication 1 ou 2, la première couche ne comprenant aucune cellule vivante.

4. Procédé selon l'une des revendications précédentes, la solution polymérisable contenant en outre des cellules.

5. Procédé selon l'une des revendications précédentes, le polymère dans la solution polymérisable étant structuré par le mouvement, avant l'étape iv).

6. Procédé selon l'une des revendications précédentes, la compression de la deuxième couche étant effectuée par centrifugation, sédimentation ou action d'une force mécanique.

7. Procédé selon l'une des revendications précédentes, les étapes de procédé iv), v) ou vi) ayant lieu l'une après l'autre et/ou simultanément.

8. Procédé selon l'une des revendications précédentes, la réalisation de l'au moins une cavité dans la matrice dermale à l'étape vi) étant effectuée pendant ou après la polymérisation à l'étape iv), de préférence pendant la polymérisation.

9. Procédé selon l'une des revendications précédentes, l'au moins une cavité étant réalisée dans la matrice dermale par piquage, découpage ou au moyen d'une empreinte.

10. Procédé selon l'une des revendications précédentes, les cavités présentant un diamètre moyen de ≥ 0,001 mm à 5 mm, de préférence de 0,005 mm à 1 mm, en particulier de préférence de 0,01 mm à 0,08 mm, tout particulièrement de préférence de 0,02 mm à 0,06 mm, en particulier de préférence de 0,05 mm à 0,04 mm.

11. Procédé selon l'une des revendications précédentes, les cavités présentant une profondeur de ≥ 0,05 mm à 10 mm, de préférence de 0,1 mm à 8 mm, en particulier de préférence de 0,15 mm à 6 mm, tout particulièrement de préférence de 0,2 mm à 4 mm, en particulier de préférence de 0,5 mm à 2,5 mm.

12. Procédé selon l'une des revendications précédentes, une, plusieurs ou toutes les cavités de la matrice dermale étant revêtues par des protéines matricielles extracellulaires avant l'apport du follicule pileux et/ou du germe de follicule pileux à l'étape b).

13. Équivalent de peau fabriqué par le procédé selon l'une des revendications 1 à 12.

14. Greffon comprenant une quantité efficace d'un équivalent de peau selon la revendication 13, en option conjointement avec des adjuvants tolérables d'un point de vue pharmaceutique.

15. Procédé pour le criblage in vitro de substances avec des propriétés régulatrices de peau et/ou de poils, comprenant les étapes :
a) mise à disposition d'un échantillon de l'équivalent de peau selon la revendication 13 ;
b) division de l'échantillon en portions ;
c) incubation d'au moins une portion avec des substances qui doivent être testées ; et
d) comparaison des paramètres des propriétés de peau et/ou de poils dans la portion avec une autre portion, laquelle n'a pas été incubée avec les substances.
